# EUROPÄISCHE PATENTSCHRIFT

(11) **EP 0 547 124 B1**
(45) Veröffentlichungstag und Bekanntmachung des Hinweises auf die Patenterteilung: **06.03.1996**
(21) Anmeldenummer: 91916297.4
(22) Anmeldetag: 06.09.1991
(51) Int. Cl.: C07C 69/73, C07C 405/00, C07F 7/18

(54) **VERFAHREN ZUR HERSTELLUNG VON E/Z-MISCHUNGEN VON 2-(6-ALKYL-BICYCLO- 3.3.0]-OCTAN-3-YLIDEN)-ESSIGSÄURE-DERIVATEN MIT ÜBERWIEGENDEM ANTEIL DES E-ISOMEREN**
PROCESS FOR MAKING E/Z MIXTURES OF 2-(6-ALKYL-BICYCLO- 3.3.0]-OCTANE-3-YLIDENE)-ACETIC ACID DERIVATIVES WITH A PREPONDERANT PROPORTION OF THE E-ISOMERS
PROCEDE DE PRODUCTION DE MELANGES E/Z DE DERIVES D'ACIDE 2-(6-ALKYL-BICYCLO- 3.3.0]-OCTAN-3-YLIDEN)-ACETIQUE A PROPORTION PREPONDERANTE D'ISOMERES E

(30) Priorität: 07.09.1990 DE 4028864
(43) Veröffentlichungstag der Anmeldung: 23.06.1993
(73) Patentinhaber: SCHERING AKTIENGESELLSCHAFT, D-13342 Berlin (DE)
(72) Erfinder: HARRE, Michael, D-1000 Berlin 41 (DE); WESTERMANN, Jürgen, D-1000 Berlin 21 (DE); NICKISCH, Klaus, D-1000 Berlin 49 (DE); REHWINKEL, Hartmut, D-1000 Berlin 44 (DE)
(86) Internationale Anmeldenummer: DE9100722
(87) Internationale Veröffentlichungsnummer: WO9204312

(56) Entgegenhaltungen:
- EP-A- 0 119 949
- EP-A- 0 303 562
- Journal of Medicinal Chemistry, Band. 29, Nr. 3, März 1986 W. Skuballa et al.: "Synthesis of a New Chemically and Metabolically Stable Prostacyclin Analogue with High and Long-Lasting Oral Activity ",
- Chemistry and Industry, Band., Dezember 1962 I. Tömösközi et al.: "Asymmetric Induction in the Wittig Reaction ",

## Beschreibung

Die Erfindung betrifft ein Verfahren zur Herstellung von E/Z-Gemischen von 2-(6-Alkyl-bicyclo-[3.3.0]-octan-3-yliden)-essigsäure-estern der Formel (I). 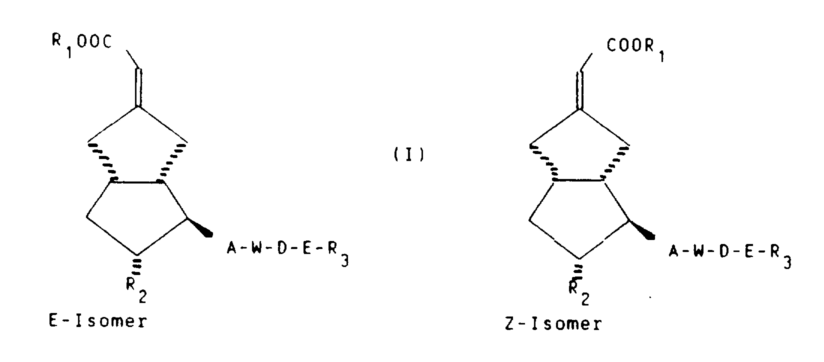 in denen der E-Anteil überwiegt.

Optisch aktives 6a-Carbacyclin und einige davon abgeleitete Verbindungen sind als stabile Analoga des natürlichen Prostacyclins (PGI₂) therapeutisch interessant. [R. Nickolson. M. H. Town und H. Vorbrüggen. Medicinal Research Reviews 5, 1, (1985)].

Bei der Synthese der 6a-Carbacycline entstehen, wenn man die obere Kette über eine Horner-Emmons-Reaktion einführt, immer ca. 50% der biologisch potenten E- und ca. 50% der fast inaktiven Z-Analoga.

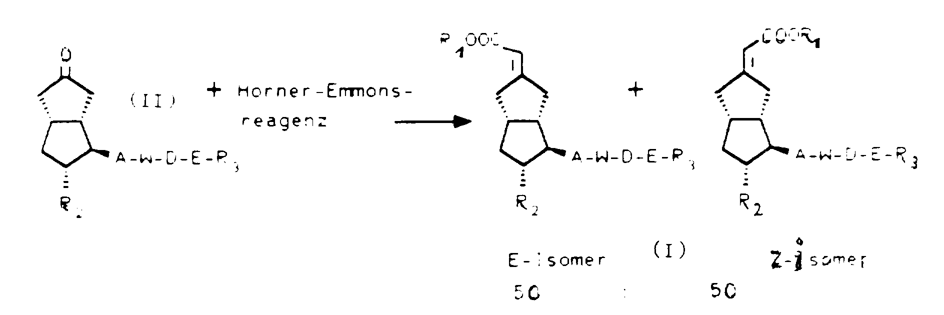

In J. Med. Chem. (1986), 29, 313 ist die o. a. Umsetzung mit einem E/Z-Verhältnis von 50:50 beschrieben.

Um die Stereoselektivität der Horner-Emmons-Reaktion zu erhöhen, wurden Umsetzungen von chiralen Phosphonsäurederivaten mit achiralen Ketonen beschrieben:

Asymmetrische Horner-Emmons-Reaktionen wurden bisher von Tömösközi und Janzo (Chem. Ind. (1962), 2085) beschrieben, die (-)-Menthylphosphonoessigsäure-P,P-diethylester mit 4-substituierten Cyclohexanonen umsetzten.

Weitere Beispiele dafür sind Bestmann et al. (Angew. Chem. 81, 751, (1969)) und Hannessian et al. (J. Am. Chem. Soc. 106, 5754 (1984)).

Erdelmeier und Gais (Angew. Chem. 98, 912 (1986)) erhielten bei einem verwandten Reaktionstyp mit chiralen Sulfoximinen an Bicyclo-[3.3.0]-octanonen unsymmetrische Olefine.

In EP 303 562 wird beschrieben, daß chirale Bicyclo-[3.3.0]-octan-3-on-derivate bei der Umsetzung mit (am Carbonesterteil) chiralen Phosphonoessigsäurederivaten erhöhte E-Anteile aufweisen.

Dabei wird die beobachtete Induktion auf den chiralen Carbonester im Phosphonat zurückgeführt.

Es wurde nun überraschend gefunden, daß einfache, nicht chirale Phosphonate bei der Umsetzung mit chiralen 6-Alkyl-bicyclo-[3.3.0]-octan-3-on-derivaten der Formel (II) ebenfalls zu erhöhtem E-Anteil führen, wenn man die Substituenten am Phosphor und am Carbonesterteil des Phosphonats (III) richtig wählt.

Die richtige Wahl der Substituenten am Phosphor bzw. am Carbonesterteil ist dabei essentiell:

Voluminöse Substituenten am Phosphor und kleine Substituenten am Carbon esterteil ergeben hohe Anteile des gewünschten E-Isomeren, wobei die Reste am Phosphor allerdings nicht zu groß gewählt werden dürfen.

Zu große Reste am Phosphor verlängern die Reaktionsdauer und geben Anlaß zu verstärkter Bildung der Iso-Verbindungen (V), bei denen die exocyclische Doppelbindung in den Ring gewandert ist.

Da im Phosphonoessigsäureester kein Chiralitätszentrum vorhanden ist, kann der erhöhte E-Anteil nur durch Substratkontrolle des Ketons (sterische Effekte) hervorgerufen worden sein.

Ein weiterer Vorteil des beschriebenen Verfahrens liegt in der Einsparung der in der EP 303 562 benutzten chiralen Alkohole, die im allgemeinen einen hohen Preis haben und nicht einfach zurückgewonnen werden können.

Das beschriebene Verfahren erreicht also die gewünschte hohe E/Z-Stereoselektivität unter Verwendung wesentlich preiswerterer, weil achiraler Reagenzien. Dadurch sind die beschriebenen Bedingungen wesentlich ökonomischer, als die oben zitierten Methoden.
Die Erfindung betrifft somit ein Verfahren zur Herstellung von E/Z-Gemischen von chiralen 2-(6-Alkyl-bicyclo-[3.3.0]-octan-3-yliden)-essigsäurederivaten der Formel (I),

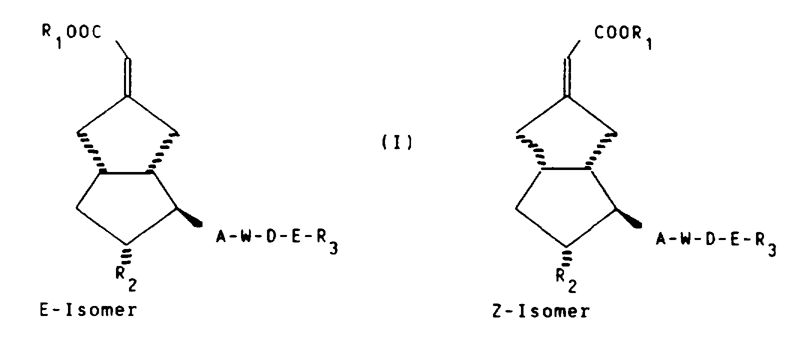

in denen das E-Isomer überwiegt und worin
- R₁: den Rest CH₃,
- R₂: eine freie oder funktionell abgewandelte, α-ständige Hydroxylgruppe,
- A: eine trans CH=CH- oder -C≡C-Gruppe,
- W: eine freie oder funktionell abgewandelte Hydroxymethylengruppe oder eine freie oder funktionell abgewandelte 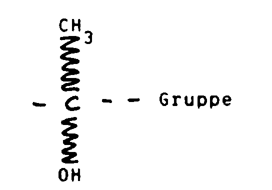 wobei die OH-Gruppe α- oder β-ständig sein kann,
- D: die Gruppe 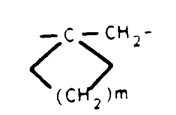 oder eine geradkettige oder verzweigte, gesättigte Alkylengruppe mit 1 bis 5 Kohlenstoffatomen, eine verzweigte oder geradkettige ungesättigte Alkylengruppe mit 2 bis 5 Kohlenstoffatomen, die gegebenenfalls durch Fluoratome substituiert sein kann,
- m: die Zahl 1,2 oder 3,
- E: eine Direktbindung, eine -C≡C-Gruppe oder eine -CR₄=CR₅- Gruppe darstellt, wobei R₄ Wasserstoff oder eine Alkylgruppe mit 1-5 Kohlenstoffatomen und R₅ Wasserstoff, Halogen oder eine Alkylgruppe mit 1 - 5 Kohlenstoffatomen bedeuten,
- R₃: eine Alkylgruppe mit 1 - 10 Kohlenstoffatomen, eine Cycloalkylgruppe mit 3 - 10 Kohlenstoffatomen oder eine gegebenenfalls substituierte Arylgruppe mit 6 - 10 Kohlenstoffatomen oder eine heterocyclische Gruppe bedeuten, dadurch gekennzeichnet, daß man eine 6-Alkyl-bicyclo-[3.3.0]-octan-3-on der Formel (II), 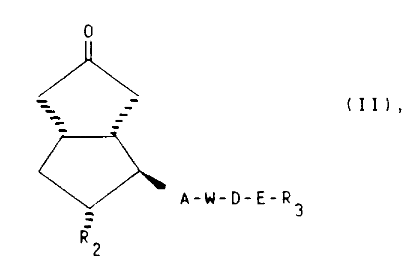 worin A, W, D, E, R₂ und R₃ die oben angegebene Bedeutung haben, mit einem P,P-Dialkoxy-phosphonoessigsäuremethylester der Formel (III),

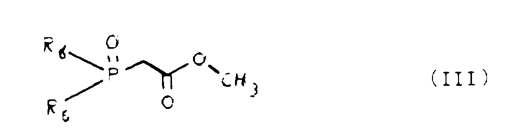

worin R₆ 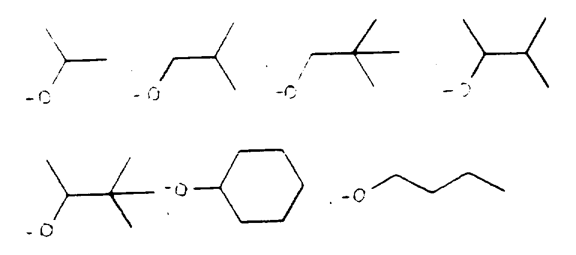 bedeutet,
in Gegenwart eines Deprotonierungmittels umsetzt.

Als Alkylgruppen R₃ kommen gerad- und verzweigtkettige, gesättigte oder ungesättigte Alkylreste, vorzugsweise gesättigte, mit 1-10, insbesondere 1-7 Kohlenstoffatomen in Frage, die durch gegebenenfalls substituiertes Aryl substituiert sein können. Beispielsweise genannt seien Methyl, Ethyl, Propyl, Butyl, Isobutyl, tert.-Butyl, Pentyl, Hexyl, Heptyl, Octyl, Butenyl, Isobutenyl, Propenyl, Pentenyl, Benzyl und p-Chlorbenzyl.

Die Cycloalkylgruppen R₃ können im Ring 3-10, vorzugsweise 3-6 Kohlenstoffatome enthalten. Die Ringe können durch Alkylgruppen mit 1-4 Kohlenstoffatomen substituiert sein. Beispielsweise seien genannt Cyclopropyl, Cyclobutyl, Cyclopentyl und Cyclohexyl.

Als heterocyclische Gruppe R 3 kommen folgende Reste in Betracht: 2-,3-oder 4-Pyridyl, 2-Furyl, 3-Furyl, 2-Thienyl, 3-Thienyl usw.

Als substituierte und unsubstituierte Arylgruppen R₃ kommen beispielsweise in Betracht: Phenyl, 1-Naphthyl und 2-Naphthyl. Bevorzugt ist die Substitution in der 4-Stellung zum Beispiel mit Fluor, Chlor, C1-C4-Alkoxy, Trifluormethyl oder Hydroxy.

Als Alkylengruppe D kommen geradkettige Alkylenreste mit 1-10 Kohlenstoffatomen oder verzweigtkettige, gesättigte und ungesättigte Alkylenreste mit 2 - 10 Kohlenstoffatomen, vorzugsweise mit 1 - 5 Kohlenstoffatomen bzw. 2 - 5 Kohlenstoffatomen in Frage, die gegebenenfalls durch Fluoratome substituiert sein können. Beispielsweise seien genannt Methylen. Fluormethylen, Ethylen, 1,2-Propylen, Ethylethylen, Trimethylen, Pentamethylen.

Die Alkylgruppen R₄ und R₅ stellen geradkettige und verzweigte gesättigte Alkylgruppen mit 1 - 5 Kohlenstoffatomen, wie sie für R₃ bereits genannt wurden, dar, R₅ als Halogen kann Chlor und Brom, vorzugsweise Chlor sein.

Die funktionell abgewandelten Hydroxygruppen in W und R₂ können durch Tetrahydropyranyl, Trimethylsilyl, tert.-Butyl-dimethylsilyl, Thexyldimethylsilyl und tert.-Butyldiphenylsilyl substituiert sein.

Die Phosphonate (III) können nach verschiedenen literaturbekannten Methoden dargestellt werden, z. B. durch Deprotonierung des käuflichen Methanphosphonsäure-diisopropylesters mit LDA oder Butyllithium und Umsetzung mit Dimethylcarbonat oder Chlorameisensäuremethylester.

Auch die Veresterung von Methanphosphonsäure-dichlorid mit den zu R₆ korrespondierenden Alkoholen mit anschließender Deprotonierung und Umsetzung mit Dimethylcarbonat oder Chlorameisensäure-methylester ist möglich.

Nach dem erfindungsgemäßen Verfahren lassen sich (E)-2-(6-Alkylbicyclo-[3.3.0]-octan-3-yliden)-essigsäureester (I) selektiv in hohen Ausbeuten darstellen. So vereinfachen sich aufwendige chromatographische Trennungen von 1:1 Gemischen, die man nach den bisherigen Verfahren erhält.

Die selektiv hergestellten E-Ester der Formel (I) ergeben bei der Reduktion, insbesondere mit DIBAH, die entsprechenden E-Allylalkohole (IV), die sich chromatographisch von den Z-Alkoholen trennen lassen.
Diese sind wichtige Zwischenprodukte für die Darstellung von chemisch und metabolisch stabilen 3-Oxacarbacyclinen wie Cicaprost [Vgl. W. Skuballa et al., J. Med. Chem. 29, 313, (1986)].

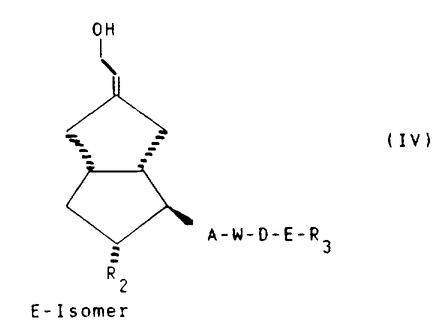

Dabei haben A, W, D, E, R₂ und R₃ die oben angegebene Bedeutung.

Bei der Umsetzung von (II) mit (III) sind die molaren Verhältnisse der Reaktionspartner variierbar.

Als vorteilhaft erweist es sich, einen Überschuß des wohlfeilen Phosphonoessigesters (III) (bezogen auf das Keton (II)) mit einem Unterschuß an Deprotonierungsmittel (bezogen auf das Phosphonat (III)) einzusetzen, da dadurch die Bildung unerwünschter Isocarbacycline (V), bei denen die exocyclische Doppelbindung in den Ring gewandert ist, unterdrückt wird.

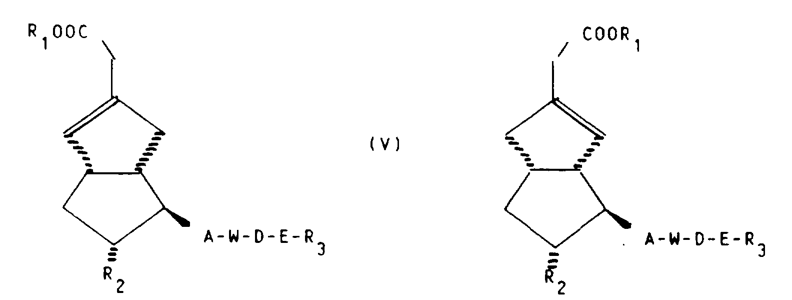

Besonders bevorzugt sind der P,P-Di-isopropoxy-phosphonoessigsäure-methylester (Phosphonat Nr. 2) und P,P-Di-neopentyloxy-phosphonoessigsäure-methylester (Phosphonat Nr. 3), die optimale E/Z-Verhältnisse bei minimalem Iso-Anteil ergeben.

Als Deprotonierungsmittel eignen sich die dem Fachmann bekannten Basen wie z. B. Alkalimetallhydride, Alkalimetallalkoholate, Alkaliamide, Kaliumtert.-butylat und Alkalisalze des Hexamethyldisilazans. Besonders vorteil-haft für die Erzielung eines hohen E/Z- Verhältnisses ist die Verwendung von Natriumhydrid.

Die Umsetzung von (II) mit (III) wird bei Temperaturen von -70°C bis +40°C, insbesondere bei -40°C bis 0°C durchgeführt.

Als Losungsmittel für die Durchführung der Reaktion eignen sich übliche Lösungsmittel wie z. B. Tetrahydrofuran, Diethylether, Hexan, 1,2-Ethylenglycoldimethylether und Toluol.

Besonders bevorzugt ist Toluol, in dem sich die höchsten E/Z-Verhältnisse einstellen und die Bildung von Isocarbacyclinen gering ist.

Zur Bestimmung des E/Z-Verhältnisses werden die E/Z-2-(6-Alkylbicyclo-[3.3.0]-octan-3-yliden)-essigsäureester nach Reduktion mit DIBAH zum Gemisch der Allylalkohole (IV) in der HPLC untersucht.

Wie die nachfolgende Tabelle zeigt, ist die Auswahl der Substituenten am Phosphor und Carbonester essentiell für einen hohen E-Anteil bei geringem Iso-Anteil: mit den Phosphonaten Nr. 2 und Nr. 3 ergeben sich hohe E/Z-Verhältnisse bei geringem Iso-Anteil, die Phosphonate Nr. 4 und Nr. 5 erzeugen nicht mehr tolerierbare Iso-Anteile.

Die nach dem erfindungsgemäßen Verfahren hergestellten (E)-Carbacyclin-Vorstufen dienen als Zwischenprodukte für die Synthese von pharmakologisch hoch wirksamen Carbacyclinen, wie z. B. Iloprost oder Cicaprost [vgl. W. Skuballa et al., J. Med. Chem. 29, 313, (1986); DE 3306123, EP 0119949]

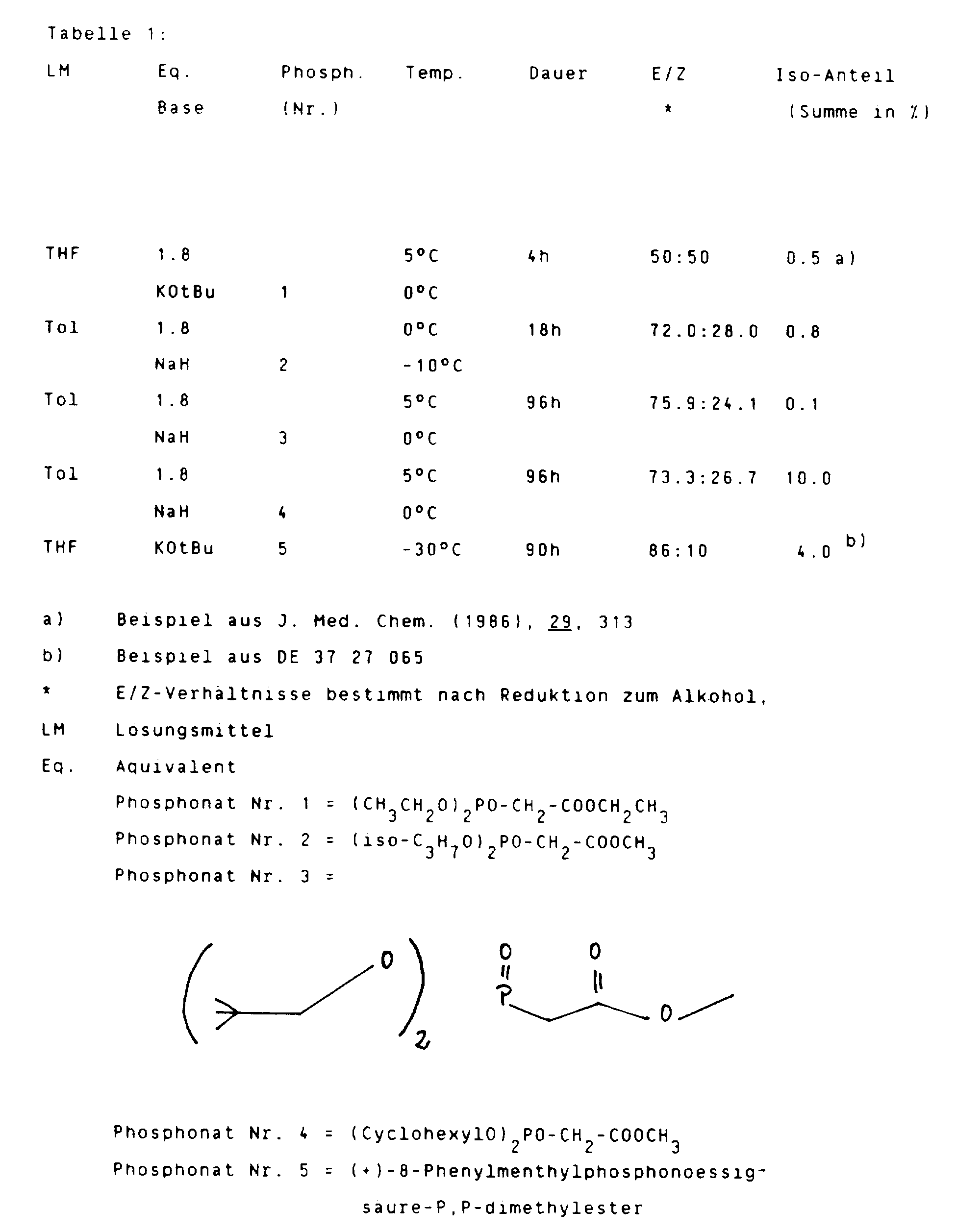

### Beispiel 1

2-<(E,Z)-(1S,5S,6S,7R)-7-tert.-Butyl-dimethylsilyloxy-6-[(3S,4S)-3-tert.-butyl-dimethylsilyloxy-4-methyl-1,6-nonadiinyl]-bicyclo[3.3.0]oct-3yliden>-essigsäure-methylester

2,28 g NaH (55 % in Weißöl) werden unter Stickstoff in einem 500 ml Dreihalskolben vorgelegt und 120 ml Toluol unter Rühren zugetropft. Bei einer Innentemperatur von +5°C wird eine Lösung von 17,5 g P,P-Di-isopropoxyphosphono-essigsäure-methylester in 100 ml Toluol zugetropft. Die Mischung wird auf -20°C abgekühlt und eine Lösung von 12,7 g (1S,2S,3R,5R)-3-tert.-Butyl-dimethylsilyloxy-2-[(3S,4S)-3-tert.-butyl-dimethyl-silyloxy-4-methyl-1,6-nonadiinyl]-bicyclo[3.3.0]octan-7-on in 20 ml Toluol zugetropft, so daß die Temperatur nicht über -15°C steigt.
Nach beendeter Zugabe wird die Lösung auf -10°C erwärmt und 18 h bei dieser Temperatur nachgerührt. Die Mischung wird mit Essigsäure auf pH 6 eingestellt, 150 ml Wasser zugegeben und 10 min nachgerührt. Die organische Phase wird abgetrennt und die wässrige Phase zweimal mit je 150 ml MTB extrahiert. Die vereinigten organischen Phasen werden mit 150 ml Wasser gewaschen, über 40 g Natriumsulfat getrocknet und eingeengt.
Das Rohprodukt wird über 670 g Kieselgel aufgereinigt.
(Hexan/Essigester 95 : 5)

- **Ausbeute :**: 13,46 g = 95,5 % d. Th.
- **Eigenschaften :**: [α]⁰ (c=1, CHCl₃₎

| | | | | |
|---|---|---|---|---|
| 589 | 578 | 546 | 436 | 365 nm |
| + 30,3 | + 32,1 | + 37,1 | + 71,6 | + 127,9 ° |

Da sich das E/Z-Verhältnis auf dieser Stufe nicht bestimmen läßt, wird der Ester zum Alkohol reduziert (siehe Beispiel 2).

### Beispiel 2

2-<(E,Z)-(1S,5S,6S,7R)-7-tert.-Butyl-dimethylsilyloxy-6-[(3S,4S)-3-tert.-butyl-dimethylsilyloxy-4-methyl-1,6-nonadiinyl]-bicyclo[3.3.0]oct-3-yliden>-ethanol

13,28 g 2-<(E,Z)-(1S,5S,6S,7R)-7-tert.-Butyl-dimethyl-silyloxy-6-[(3S,4S)-3-tert.butyl-dimethylsilyloxy-4-methyl-1,6-nonadiinyl]-bicyclo[3.3.0]oct-3-yliden>-essigsäure-methylester werden unter Stickstoff in 259 ml Toluol gelöst. Die Lösung wird auf -30°C abgekühlt und bei dieser Temperatur- 41,3 ml Dibah-T zugegeben. Die gelbliche Lösung wird 2,5 Stunden bei - -30°C nachgerührt. Es werden 15 ml Wasser zugetropft und bei 20-22°C 30 min nachgerührt. Das ausgefallene Aluminiumhydroxid wird abfiltriert und der Rückstand zweimal mit je 100 ml Methyl-tert.-butylether nachgewaschen. Das Filtrat wird eingeengt.

### Ausbeute: 12,8 g = 90,8 % der Theorie

E/Z-Verhältnis im Rohprodukt nach HPLC [Lichrosorb DIOL, 250 mm, 5µ, Hexan/ Methyl-tert.-butylether 9+1, 205 nm]:
2-<(E)-(1S,5S,6S,7R)-7-tert.-Butyl-dimethylsilyloxy-6-[(3S,4S)-3-tert.-butyl-dimethylsilyloxy-4-methyl-1,6-nonadiinyl]-bicyclo[3.3.0]oct-3-yliden>-ethanol zu 2-<(Z)-(1S,5S,6S,7R)-7-tert.-Butyl-dimethylsilyloxy-6-[(3S,4S)-3-tert.-butyl-dimethylsilyloxy-4-methyl-1,6-nonadiinyl]-bicyclo[3.3.0]oct-3-yliden>-ethanol = 72:28
Iso-Verbindungen Summe 0,8 %

Nach chromatographischer Auftrennung werden folgende Eigenschaften bestimmt: 2-<(E)-(1S,5S,6S,7R)-7-tert.Butyl-dimethylsilyloxy-6-[(3S,4S)-3-tert.butyl dimethylsilyloxy-4-methyl-1,6-nonadiinyl]-bicyclo[3.3.0]oct-3-yliden>ethanol als klares, farbloses Öl

| [α]⁰ (c=1, CHCl₃) | | | | |
|---|---|---|---|---|
| 589 | 578 | 546 | 436 | 365 nm |
| + 23,2 | +24,0 | +27,6 | +49,0 | +83,1 ° |

2-<(Z)-[1S,5S,6S,7R]-7-tert.Butyl-dimethylsilyloxy-6-[(3S,4S)-3-tert.-butyl-dimethylsilyloxy-4-methyl-1,6-nonadiinyl]-bicyclo[3.3.0]oct-3-yliden>-ethanol als klares, farbloses Öl

| [α]⁰ (c=1, CHCl₃) | | | | |
|---|---|---|---|---|
| 589 | 578 | 546 | 436 | 365 nm |
| + 12,3 | +12,8 | +15,0 | +29,1 | +83,1 ° |

### Beispiel 3

2-<(E,Z)-(1S,5S,6S,7R)-7-tert.-Butyl-dimethylsilyloxy-6-[(3S,4S)-3-tert.-butyl-dimethylsilyloxy-4-methyl-1,6-nona-diinyl]-bicyclo[3.3.0]oct-3-yliden>-essigsäure-methylester

Unter Stickstoff werden 38 mg NaH (55 % in Weißöl) in einem 10 ml Kolben vorgelegt und 2 ml Toluol zugegeben. Bei einer Innentemperatur von +5°C wird eine Losung von 423 mg P,P-Di-neopentyloxy-phosphonoessigsäure-methylester in 2 ml Toluol zugegeben. Die Mischung wird auf -20°C abgekühlt und eine Lösung von 250 mg (1S,2S,3R,5R)-3-tert.-Butyl-dimethylsilyloxy-2-[(3S,4S)-3-tert.-butyl-dimethylsilyloxy-4-methyl-1,6-nonadiinyl]-bicyclo[3.3.0]octan-7-on in 2 ml Toluol zugegeben, so daß die Temperatur nicht über -15°C steigt. Nach beendeter Zugabe wird die Lösung auf 0°C erwärmt und 4 Tage bei dieser Temperatur gerührt. Die Mischung wird mit Essigsäure auf pH 6 eingestellt, 5 ml Wasser zugegeben und 10 min nachgerührt. Die organische Phase wird abgetrennt und die wässrige Phase mit 15 ml MTB extrahiert. Die vereinigten organischen Phasen werden mit 15 ml Wasser gewaschen, über 4 g Natriumsulfat getrocknet und eingeengt. Das Rohprodukt wird über 35 g Kieselgel aufgereinigt.
(Hexan/Essigester 95 : 5)

Da sich das E/Z Verhältnis auf dieser Stufe nicht bestimmen läßt, wird der Ester zum Alkohol reduziert (siehe Beispiel 4).

### Beispiel 4

2-<(E,Z)-(1S,5S,6S,7R)-7-tert.-Butyl-dimethylsilyloxy-6-[(3S,4S]-3-tert.-butyl-dimethylsilyloxy-4-methyl-1,6-nonadiinyl]-bicyclo[3.3.0]oct-3-yliden>-ethanol

Die Verbindung aus Beispiel 3 wird reduziert, wie in Beispiel 2 beschrieben und ergibt:

E/Z Verhältnis im Rohprodukt nach HPLC [Lichrosorb DIOL, 250 mm, 5µ, Hexan/ Methyl-tert.-butylether 9+1, 205 nm]:
2-<(E)-(1S,5S,6S,7R)-7-tert.-Butyl-dimethylsilyloxy-6-[(3S,4S)-3-tert.-butyl-dimethylsilyloxy-4-methyl-1,6-nonadiinyl]-bicyclo[3.3.0]oct-3-yliden>-ethanol zu

2-<(Z)-(1S,5S,6S,7R)-7-tert.-Butyl-dimethylsilyloxy-6-[(3S,4S)-3-tert.butyl-dimethylsilyloxy-4-methyl-1,6-nonadiinyl]-bicyclo[3.3.0]oct-3-yliden>-ethanol 75,9 : 24,1
Iso-Verbindungen Summe 0,1 %

## Patentansprüche

1. Verfahren zur Herstellung von E/Z-Gemischen von chiralen 2-(6-Alkyl-bicyclo-[3.3.0]-octan-3-yliden)-essigsäureestern der Formel I, in denen der E-Anteil überwiegt, 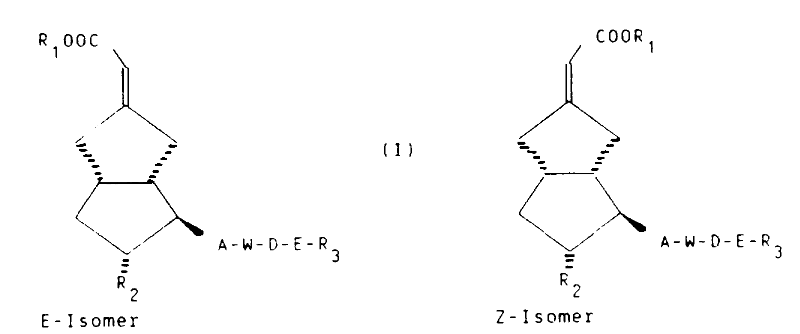 worin
R₁ den Rest CH₃
R₂ eine freie oder funktionell abgewandelte, α-ständige Hydroxylgruppe,
A eine trans-CH=CH- oder -C≡C-Gruppe,
W eine freie oder funktionell abgewandelte Hydroxymethylengruppe oder eine freie oder funktionell abgewandelte 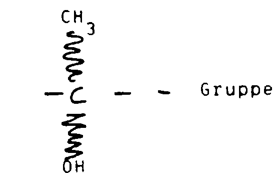 wobei die OH-Gruppe α- oder β-ständig sein kann,
D die Gruppe 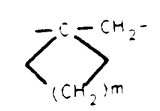 oder
eine geradkettige, gesättigte Alkylengruppe mit 1 - 5 Kohlenstoffatomen, eine verzweigte oder geradkettige ungesättigte Alkylengruppe mit 2 bis 5 Kohlenstoffatomen, die gegebenenfalls durch Fluoratome substituiert sein können,
m die Zahl 1,2 oder 3,
E eine Direktbindung, eine -C≡C-Gruppe oder eine -CR₄=CR₅-Gruppe, wobei R₄ Wasserstoff oder eine Alkylgruppe mit 1 - 5 Kohlenstoffatomen und R₅ Wasserstoff, Halogen oder eine Alkylgruppe mit 1 - 5 Kohlenstoffatomen bedeuten,
R₃ eine Alkylgruppe mit 1 - 10 Kohlenstoffatomen, eine Cycloalkylgruppe mit 3 - 10 Kohlenstoffatomen oder eine gegebenenfalls substituierte Arylgruppe mit 6 - 10 Kohlenstoffatomen oder eine heterocyclische Gruppe bedeuten, dadurch gekennzeichnet, daß man ein 6-Alkyl-bi-cyclo-[3.3.0]-octan-3-on der Formel (II), 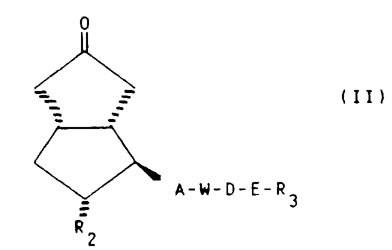 worin A, W, D, E, R₂ und R₃ die oben angegebene Bedeutung haben, mit einem P,P-Dialkoxy-phosphonoessigsäuremethylester der Formel (III), 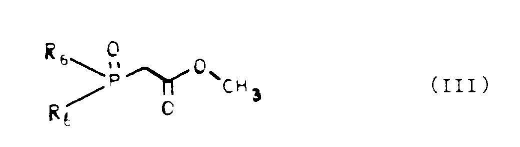 worin
R₆ 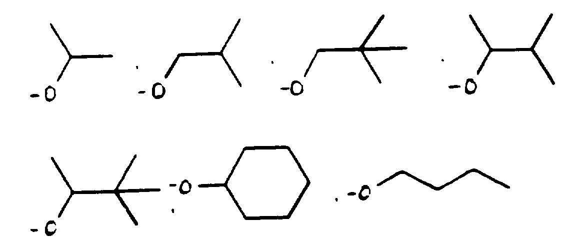 bedeuten, in Gegenwart eines Deprotonierungsmittels umsetzt.

## Claims

1. Process for the preparation of E/Z mixtures of chiral 2-(6-alkyl-bicyclo[3.3,0]octan-3-ylid-ene]acetic acid esters of the formula I in which the E content predominates 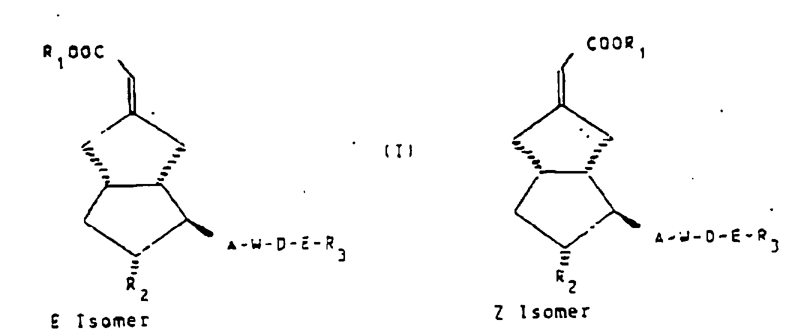 in which
R₁ is the radical CH₃,
R₂ is a free or functionally modified hydroxy group in the α-configuration,
A is a trans-CH=CH- or -C≡C- group,
W is a free or functionally modified hydroxymethylene group or a free or functionally modified 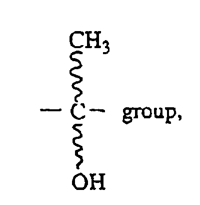 in which the OH group may be in the α- or the β-configuration,
D is the group 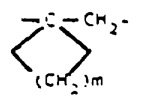 or
a straight-chained, saturated alkylene group having 1 - 5 carbon atoms, or a branched or straight-chained unsaturated alkylene group having from 2 to 5 carbon atoms, each of which may optionally be substituted by fluorine atoms,
m is the number 1, 2 or 3,
E is a direct bond, a -C≡C- group or a -CR₄=CR₅- group in which R₄ is hydrogen or an alkyl group having 1 - 5 carbon atoms and R₅ is hydrogen, halogen or an alkyl group having 1 - 5 carbon atoms,
R₃ is an alkyl group having 1 - 10 carbon atoms, a cycloalkyl group having 3 - 10 carbon atoms or an optionally substituted aryl group having 6 - 10 carbon atoms or a heterocyclic group,
characterised in that a 6-alkyl-bicyclo[3.3.0]octan-3-one of the formula (II) 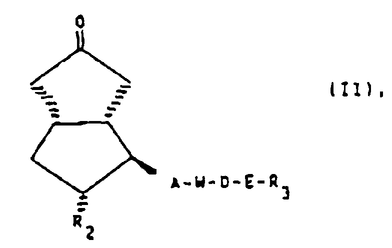 in which A, W, D, E, R₂ and R₃ have the meaning given above, is reacted with a P,P-dialkoxy-phosphonoacetic acid methyl ester of the formula (III) 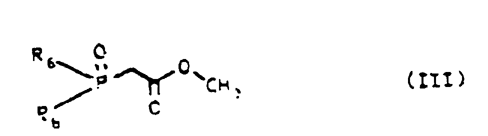 in which
R₆ is 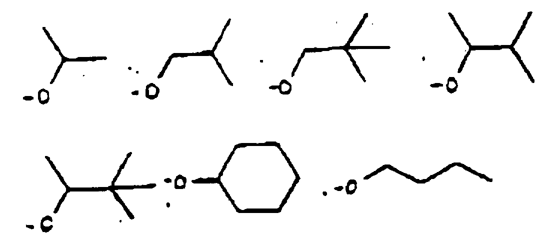 in the presence of a deprotonating agent.

## Revendications

1. Procédé pour la préparation de mélanges E/Z d'esters d'acide 2-(6-alkyl-bicyclo-[3.3.0]-octan-3-ylidène)-acétique de formule I dans lesquels la portion de E est prépondérante. 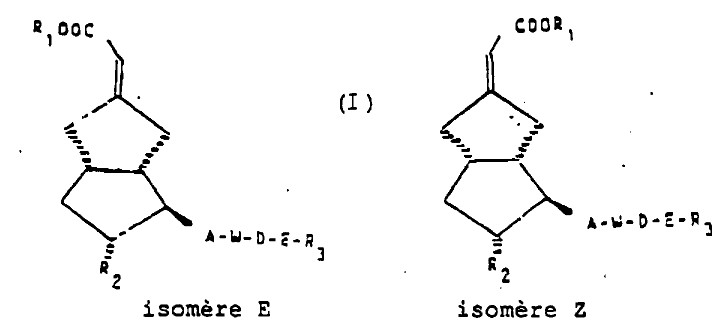 où
R₁ représente le radical CH₃
R₂ un groupe hydroxyle libre ou fonctionnellement modifiée en position α
A un groupe trans-CH=CH- ou -C≡C-
W un groupe hydroxyle libre ou fonctionnellement modifié ou un groupe libre ou fonctionnellement modifié 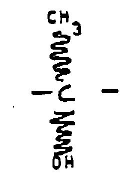 le groupe OH pouvant être en position α ou β
D le groupe 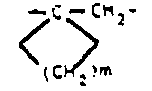 ou
un groupe alkyléne linéaire, saturée, avec 1 à 5 atomes de carbone, un groupe alkylène ramifié ou linéaire insaturé avec 2 à 5 atomes de carbone qui peut être éventuellement substitué par des atomes de fluor
m vaut 1, 2 ou 3
E représente une liaison directe, un groupe -C≡C- ou un groupe -CR₄=CR₅, R₄ signifiant l'hydrogène ou un groupe alkyle avec 1 à 5 atomes de carbone et R₅ l'hydrogène, un halogène ou un groupe alkyle avec 1 à 5 atomes de carbone,
R₃ représente un groupe alkyle avec 1 à 10 atomes de carbone, un groupe cycloalkyle avec 3 à 10 atomes de carbone ou un groupe alkyle avec 6 à 10 atomes de carbone, éventuellement substitué, ou un groupe hétérocyclique, caractérisé en ce qu'on fait réagir une 6-(alkyl-bicyclo-[3.3.0]-octan-3-one de formule (II) 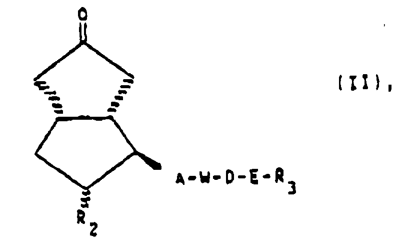 où A, W, D, E, R₂ et R₃ ont des significations données ci-dessus avec un P,P-dialcoxy-phosphonoacétate de méthyle de formule (III) 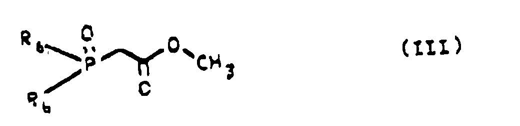 où
R₆ représente 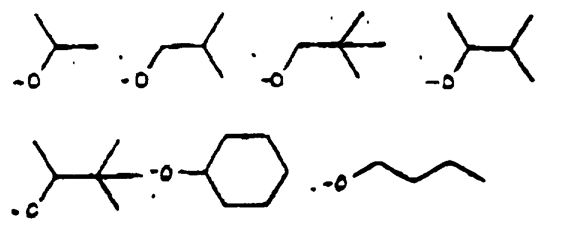 en présence d'un agent de déprotonation.
